Europäisches Patentamt

European Patent Office

Office européen des brevets

(19)

(11) Veröffentlichungsnummer : **0 130 288**
**B1**

(12) **EUROPÄISCHE PATENTSCHRIFT**

(45) Veröffentlichungstag der Patentschrift :
02.03.88

(21) Anmeldenummer : 84103776.5

(22) Anmeldetag : 05.04.84

(51) Int. Cl.⁴ : **C 12 N 9/04**, **C 12 P 7/40**,
**C 12 P 11/00**

(54) **Mikrobiologisch hergestellte D-2-Hydroxy-4-methylpentansäure-Dehydrogenase, Verfahren zu ihrer Gewinnung und ihre Verwendung.**

(30) Priorität : 07.06.83 DE 3320495

(43) Veröffentlichungstag der Anmeldung :
09.01.85 Patentblatt 85/02

(45) Bekanntmachung des Hinweises auf die Patenterteilung : 02.03.88 Patentblatt 88/09

(84) Benannte Vertragsstaaten :
BE CH DE FR GB LI NL SE

(56) Entgegenhaltungen :
EP-A- 0 000 560
EP-A- 0 024 547
EP-A- 0 103 210
DE-A- 2 841 414
CHEMICAL ABSTRACTS, Band 99, Nr. 23, 5. Dezember 1983, Seiten 609-610, Nr. 193133w, Columbus, Ohio, US; W. HUMMEL u.a.: "Large scale production of D-lactate dehydrogenase for the stereospecific reduction of pyruvate and phenylpyruvate"
CHEMICAL ABSTRACTS, Band 100, Nr. 25, 28. Juni 1984, Seiten 231-232, Nr. 205453e, Columbus, Ohio, US; H. SCHUETTE u.a.: "L-2-hydroxyisocaproate dehydrogenase - a new enzyme from Lactobacillus confusus for the stereospecific reduction of 2-keto-carboxylic acids"

(73) Patentinhaber : Degussa Aktiengesellschaft
Weissfrauenstrasse 9
D-6000 Frankfurt am Main 1 (DE)

Gesellschaft für Biotechnologische Forschung mbH (GBF)
Mascheroder Weg 1
D-3300 Braunschweig-Stöckheim (DE)

(72) Erfinder : Leuchtenberger, Wolfgang, Dr. Dipl.-Chem.
Geschwister-Scholl-Strasse 1
D-6454 Bruchköbel (DE)
Erfinder : Hummer, Werner, Dr.
Glückstrasse 3
D-3300 Braunschweig (DE)
Erfinder : Kula, Maria-Regina, Dr.
Forstweg 15
D-3340 Wolfenbüttel (DE)
Erfinder : Schütte, Horst
Nordring 29a
D-3332 Salzgitter 51 (DE)

## Beschreibung

Gegenstand der Erfindung ist eine mikrobiologisch hergestellte D-2-Hydroxy-4-methylpentansäure-Dehydrogenase, gekennzeichnet durch folgende Eigenschaften :

a) katalysiert in Abhängigkeit von dem Coenzym Nicotinamid-adenin-dinucleotid (NAD$^+$) die Dehydrogenierung von D-2-Hydroxy-4-methylpentansäure zu 2-Keto-4-methylpentansäure,

b) katalysiert in Abhängigkeit von dem Coenzym NAD$^+$ die Dehydrogenierung weiterer D-2-Hydroxycarbonsäuren zu den entsprechenden 2-Ketocarbonsäuren und zeigt insbesondere zusätzliche Aktivität gegenüber D-2-Hydroxy-pentansäure, D-2-Hydroxy-hexansäure, D-2-Hydroxy-octansäure, D-2-Hydroxy-4-(methylmercapto)-buttersäure und D-2-Hydroxy-3-phenylpropionsäure,

c) katalysiert die stereoselektive Reduktion der 2-Keto-4-methylpentansäure zu D-2-Hydroxy-4-methylpentansäure mit NADH als Coenzym,

d) katalysiert die stereoselektive Reduktion weiterer 2-Ketocarbonsäuren, insbesondere von 2-Ketobuttersäure, 2-Ketopentansäure, 2-Keto-3-methylbuttersäure, 2-Ketohexansäure, 2-Keto-3-methylpentansäure, 2-Ketooctansäure, 2-Keto-3-mercaptopropionsäure, 2-Keto-4-(methylmercapto)-buttersäure und 2-Keto-3-phenylpropionsäure zu den entsprechenden D-2-Hydroxycarbonsäuren mit NADH als Coenzym,

e) einen optimalen pH-Bereich für die Dehydrogenierungsreaktion von 7,5 bis 9,0,

f) einen optimalen pH-Bereich für die Reduktionsreaktion von 5,5 bis 7,0 und

g) einen pH-Stabilitätsbereich von 3,5 bis 10

und erhältlich durch Züchtung eines der Stämme Lactobacillus casei ssp. pseudoplantarum (DSM 20008) Lactobacillus casei ssp. alactosus (DSM 20020), Lactobacillus casei ssp. rhamnosus (DSM 20178), Leucostonoc oenos (DSM 20252), Leucostonoc lactis (DSM 20202) oder Leucostonoc mesenteroides (DSM 20343) und Gewinnung daraus.

Ein weiterer Gegenstand der Erfindung ist ein Verfahren zur Gewinnung der neuen D-2-Hydroxy-4-methylpentansäure-Dehydrogenase, welches dadurch gekennzeichnet ist, daß man Lactobacillus casei ssp. pseudoplantarum (DSM 20008), Lactobacillus casei ssp. alactosus (DSM 20020), Lactobacillus casei ssp. rhamnosus (DSM 20178), Leuconostoc oenos (DSM 20252), Leuconostoc lactis (DSM 20202) oder Leuconostoc mesenteroides (DSM 20343) in einem wässrigen Nährmedium züchtet, das eine Quelle für Kohlenstoff, Stickstoff, Vitamine, gegebenenfalls Wuchsstoffe, sowie Mineralsalze enthält und zu Beginn der Züchtung einen pH-Wert von 6,5 aufweist, nach beendeter Züchtung die Zellen durch Zentrifugation erntet, sie in einer auf pH 7 gepufferten Suspension aufschließt und das Enzym aus dem Rohextrakt in an sich bekannter Weise weiter anreichert.

Gegenstand der Erfindung ist schließlich auch die Verwendung des neuen Enzyms zur enzymatisch katalysierten Umwandlung von D-2-Hydroxy-4-methylpentansäure, D-2-Hydroxy-pentansäure, D-2-Hydroxy-hexansäure, D-2-Hydroxy-octansäure, D-2-Hydroxy-4-(methylmercapto)-buttersäure oder D-2-Hydroxy-3-phenylpropionsäure in die entsprechenden 2-Ketocarbonsäuren bzw. von 2-Keto-4-methylpentansäure, 2-Ketobuttersäure, 2-Ketopentansäure, 2-Keto-3-methylbuttersäure, 2-Ketohexansäure, 2-Keto-3-methylpentansäure, 2-Ketooctansäure, 2-Keto-3-mercaptopropionsäure, 2-Keto-4-(methylmercapto)-buttersäure und 2-Keto-3-phenylpropionsäure in die entsprechenden D-2-Hydroxycarbonsäuren.

Für die stereospezifische Reduktion von 2-Ketocarbonsäuren zu D-2-Hydroxycarbonsäuren sind aus der Literatur folgende von NADH abhängige Enzyme bekannt : D-Lactat-Dehydrogenase (E.C. 1.1.1.28). Das neue Enzym unterscheidet sich von der D-Lactat-Dehydrogenase, da die D-2-Hydroxy-4-methylpentansäure-Dehydrogenase Pyruvat nicht als Substrat akzeptiert. Ferner ist eine D-2-Hydroxy-fettsäure-Dehydrogenase (E.C. 1.1.1.98) aus Rattennieren isoliert worden, wo es an der β-Oxidation von langkettigen Fettsäuren beteiligt ist. Für dieses Enzym ist jedoch nur 2-Hydroxystearat als Substrat beschrieben.

Das erfindungsgemäße Enzym kann aus verschiedenen, bei der Deutschen Sammlung von Mikroorganismen in Göttingen erhältlichen Mikroorganismen, nämlich Lactobacillus casei ssp. pseudoplantarum (DSM 20008), Lactobacillus casei ssp. rhamnosus (DSM 20178), Lactobacillus casei ssp. alactosus (DSM 20020), Leuconostoc oenos (DSM 20252), Leuconostoc mesenteroides (DSM 20343) und Leuconostoc lactis (DSM 20202) gewonnen werden. Diese Stämme wurden bei einem Screening unter 45 Stämmen der Gattungen Lactobacillus und Leuconostoc als D-2-Hydroxy-4-methylpentansäure-Dehydrogenase-Produzenten identifiziert.

Das neue Enzym wird durch einen photometrischen Test gemessen. Der Testansatz (3,00 ml) enthält 0,1 M Phosphatpuffer für pH 7,0, 0,235 mM NADH, 0,79 mM 2-Keto-4-methylpentansäure und limitierende Mengen Enzym. Gemessen wird die Abnahme der NADH-Extinktion bei 340 nm bei 30 °C, abgezogen wird ein Nullwert, den man erhält, wenn der Testansatz ohne die 2-Ketocarbonsäure inkubiert wird. Die Enzymaktivität wird in internationalen Einheiten (U) angegeben, wobei 1 U den Verbrauch von 10$^{-6}$ Mol NADH · min$^{-1}$ · ml$^{-1}$ katalysiert. Für die Berechnung wird ein molarer Extinktionskoeffizient von NADH bei 340 nm von 6,22 · 10$^3$ zugrundegelegt. Bei dem beschriebenen Testansatz werden sowohl D-2-Hydroxy-4-methylpentansäure-Dehydrogenasen als auch L-2-Hydroxy-4-methylpentansäure-Dehydrogenasen erfaßt. Positive Stämme werden nochmals mit der chiralen, kommerziell nicht erhältlichen D-2-

2

Hydroxy-4-methylpentansäure als Substrat getestet. Gegenüber der D-2-Hydroxycarbonsäure zeigt nur die erfindungsgemäße D-2-Hydroxy-4-methylpentansäure-Dehydrogenase Aktivität, während die L-2-Hydroxy-4-methylpentansäure-Dehydrogenase inaktiv ist. Ein weiterer Beweis für die Stereospezifität der katalytischen Umsetzung wird durch Versuche im Membranreaktor erbracht, die weiter unten detailliert beschrieben werden.

Die im Rohextrakt der einzelnen D-2-Hydroxy-4-methylpentansäure-Dehydrogenase produzierenden Mikroorganismen gemessenen spezifischen Aktivitäten und Volumenaktivitäten sind in Tabelle 1 aufgeführt. Die Züchtung der Mikroorganismen erfolgte jeweils in dem im Katalog der Deutschen Sammlung von Mikroorganismen angegebenen Nährmedium. Aktivitätsmessungen mit zwei weiteren 2-Ketocarbonsäuren als Substrat zeigten, daß das Enzym aus Lactobacillus casei ssp. pseudoplantarum (DSM 20008) das breiteste Substratspektrum besitzt und daher besonders geeignet ist, eine Reihe von D-2-Hydroxycarbonsäuren mit guter Aktivität umzusetzen (siehe Tabelle 1).

(Siehe Tabelle 1 Seite 4 f.)

Im folgenden wird die Gewinnung des neuen Enzyms aus Lactobacillus casei ssp. pseudoplantarum (DSM 20008) beispielhaft beschrieben :

1. Züchtung des Mikroorganismus

Zur Züchtung wird Lactobacillus casei ssp. pseudoplantarum (DSM 20008) in folgendem Medium angezogen :

| | | |
|---|---|---|
| Glukose | 20 | g |
| Hefeextrakt | 10 | g |
| Fleischextrakt | 0,5 | g |
| Natriumacetat | 5 | g |
| $K_2HPO_4$ | 2 | g |
| $MgSO_4$ | 0,2 | g |
| $MnSO_4$ | 0,05 | g |

deinoisiertes Wasser auf 1 l.

Der pH-Wert dieser Lösung wird auf 6,5 eingestellt, dann wird für 15 Minuten bei 121 °C (2 bar) sterilisiert. Zur Anzucht werden 5 ml Medium im Reagenzglas mit einer Öse voll Lactobacillus casei ssp. pseudoplantarum vom Stichagar-Röhrchen beimpft und 16 bis 20 Stunden bei 30 °C bebrütet. 4 ml der gewachsenen Kultur werden dann als Animpfkultur für 200 ml Medium (in 500 ml-Erlenmeyer-Kolben) verwendet. Nach 20 bis 24 Stunden können diese 200 ml als Vorkultur für einen 10 l-Fermenter verwendet werden, damit können dann gegebenenfalls 200 l und 5 000 l angeimpft werden. Der pH-Wert, der im Verlauf des Wachstums absinkt, wird in den Fermentern mit konzentriertem Ammoniak bei 5,0 gehalten. Die Kultivierung im Fermenter erfolgt unter leichtem Rühren unter Begasen mit Stickstoff. Gegen Ende der logarithmischen Wachstumsphase wird die Kultur abgekühlt und die Zellen durch Zentrifugation geerntet. Dabei werden 45 kg abgeschleuderte Zellmasse aus 4 500 l Kultur erhalten. Die Biomasse kann bei — 20 °C ohne größere Verluste an Aktivität für einige Monate zwischengelagert werden.

2. Isolierung und Reinigung des Enzyms

a) Rohextrakt

500 g Lactobacillus casei ssp. pseudoplantarum (DSM 20008) — Feuchtmasse werden in 50 mM Phosphatpuffer für pH 7,0, der 0,1 % (v/v) 2-Mercaptoethanol enthält, suspendiert. Das Endvolumen von 1 250 ml entspricht einer 40 %igen Zellsuspension. Die Zellen werden entweder in einer kontinuierlich arbeitenden Glasperlen-Kugelmühle oder einem Hochdruckhomogenisator aufgeschlossen. Als Glasperlen-Kugelmühle wird hier die Dynomühle Type KDL der Firma Bachofen eingesetzt.

Der 600 ml fassende Mahlbehälter wird mit 0,25 bis 0,5 mm großen Glasperlen gefüllt, so daß sich ein Schüttelvolumen von 510 ml ergibt (85 %). Der Aufschluß wird bei einer Rührwellendrehzahl von 3 000 UPM und einer Durchflußrate von 5 l/h durchgeführt. Der Kühlmantel des Mahlbehälters sowie das Rührwellenlager werden während des Laufes mit Ethylenglycollösung von — 20 °C gekühlt, um eine Erwärmung des Produktes weitgehend zu vermeiden. Nach drei Durchläufen ist ein Desintegrationsgrad von > 90 % erreicht. Der pH-Wert der Suspension fällt während der Homogenisation auf 6,1 ab und wird mit Kalilauge auf pH 7,0 zurückgestellt. Als Hochdruckhomogenisator kann z. B. das Gerät Manton Gaulin Typ 15 M-8TA (A. P. V. Schröder GmbH, Lübeck) eingesetzt werden. Die Zellsuspension wird dabei bei 550 bar unter Verwendung eines Homogenisationsventils speziell für den Mikroorganismenaufschluß und einer Durchflußrate von 54 l/h aufgeschlossen. Nach zwei Durchläufen ist ein Desintegrationsgrad von > 90 % erreicht. Nach jedem Durchlauf wird das Homogenat mittels eines Durchflußkühlers auf Temperaturen unter + 10 °C abgekühlt, um eine Erwärmung des Produktes über 20 °C zu vermeiden.

Tabelle 1 : Enzymaktivität im Rohextrakt verschiedener Mikroorganismen

| Stamm | DSM Nummer | Enzymaktivitäten mit Substrat | | | | | |
|---|---|---|---|---|---|---|---|
| | | 1 | | 2 | | 3 | |
| | | U/mg | U/l | U/mg | U/l | U/mg | U/l |
| Leuconostoc oenos | 20 252 | 18,13 | 700 | 1,35 | 52 | 1,77 | 69 |
| Leuconostoc mesenteroides | 20 343 | 1,67 | 101 | 2,86 | 173 | 9,30 | 563 |
| Leuconostoc lactis | 20 202 | 1,03 | 53 | 0,45 | 23 | 0 | 0 |
| Lactobacillus casei ssp. pseudoplantarum | 20 008 | 0,58 | 108 | 1,88 | 316 | 7,25 | 1216 |
| Lactobacillus casei ssp. rhamnosus | 20 178 | 0,38 | 43 | 1,15 | 128 | 5,93 | 660 |
| Lactobacillus casei ssp. alactosus | 20 020 | 0,49 | 42 | 0,94 | 180 | 3,11 | 594 |

Substrat : 1 = 2-Keto-4-methylpentansäure
2 = 2-Keto-4-(methylmercapto)-buttersäure
3 = 2-Keto-3-phenylpropionsäure
angegeben sind die spezifischen Enzymaktivitäten (U/mg Protein) und Volumenaktivitäten (U/l Medium)

Der pH-Wert der Suspension wird nach dem Aufschluß auf pH 7,0 eingestellt.

b) Flüssig-Flüssig-Verteilung

Mit dem ersten Verteilungsschritt sollen die Zellbruchstükke aus dem Rohextrakt gemäß DE-PS 26 39 129 abgetrennt werden. Zu diesem Zweck wird ein wässriges Zweiphasensystem hergestellt, welches 20 % (w/w) Polyethylenglycol 1 500, 8 % (w/w) Phosphatpuffer für pH 7,0 und 1 250 ml Rohextrakt in einem 2,5 kg-System enthält. Um das Gleichgewicht der Verteilung zu erreichen, wird das Zweiphasensystem eine Stunde lang gerührt und anschließend durch Zentrifugation getrennt. Größere Volumina werden zweckmäßigerweise kontinuierlich mit einem Tellerseparator, z. B. vom Typ Gyrotester B von α-Laval oder Westfalia, Typ SAOH-205, separiert. Die Oberphase (1 440 ml) enthält praktisch die gesamte Aktivität (Ausbeute 99 %) an D-2-Hydroxy-4-methylpentansäure-Dehydrogenase. Die Unterphase enthält Zellbruchstücke und wird verworfen. Die enzymhaltige Oberphase wird mit 2 % (w/v) Polyethylenglycol 10 000, 8 % Phosphatpuffer für pH 6,0 und 0,4 M Natriumchlorid, berechnet auf ein Endvolumen von 2 880 ml, versetzt und eine Stunde gerührt. Das sich ausbildende Polyethylenglycol/Salz-System läßt man in einem Standzylinder absetzen, die Trennung ist nach etwa einer Stunde vollständig. Bei diesem Verteilungsschritt wird die D-2-Hydroxy-4-methylpentansäure-Dehydrogenase in die salzhaltige Unterphase extrahiert. Die Trennung der Phasen erfolgt durch Ablaufenlassen der Unterphase.

c) Diafiltration

Die Unterphase wird mit einer Amicon Hohlfaserpatrone (Typ H1P10) konzentriert und durch Zugabe von Phosphatpuffer für pH 7,0 auf eine Salzkonzentration von 50 mM diafiltriert.

d) DEAE-Cellulose-Chromatographie

Das konzentrierte und diafiltrierte Enzym wird auf eine 5 × 30 cm Säule aufgepumpt, die mit Whatman-Cellulose DE 52 gepackt ist. Die DEAE-Cellulose ist gegen einen Puffer äquilibriert worden, der 50 mM Phosphatpuffer für pH 7,0 und 0,1 % (v/v) 2-Mercaptoethanol enthält. Die Säule wird zunächst mit etwa 2 l Startpuffer nachgewaschen und das Enzym anschließend mit einem linearen Gradienten (2 × 2 l) von 0 bis 0,25 M Natriumchlorid in Startpuffer eluiert. Die D-2-Hydroxy-4-methylpentansäure-Dehydrogenase eluiert mit ca. 0,1 M Natriumchlorid. Die aktiven Fraktionen werden durch Ultrafiltration konzentriert und bei 4 °C aufbewahrt. Die Aufreinungsschritte sind in Tabelle 2 zusammengefaßt.

Tabelle 2 : (Siehe Tabelle 2 Seite 6 f.)

## Tabelle 2

### Reinigung von D-2-Hydroxy-4-methylpentansäure-Dehydrogenase

| Reinigungsschritt | Volumen ml | Protein mg | Totalaktivität U | Spez. Aktivität U/mg | Ausbeute % | Anreicherung -fach |
|---|---|---|---|---|---|---|
| Rohextrakt | 1 250 | 30 500 | 18 625 | 0,61 | 100 | 1 |
| Oberphase I | 1 440 | 6 120 | 18 438 | 3,01 | 99 | 4,9 |
| Unterphase II | 1 060 | 3 795 | 15 857 | 4,18 | 85 | 6,9 |
| Diafiltration | 187 | 3 150 | 15 064 | 4,78 | 81 | 7,8 |
| DEAE-Cellulose | 493 | 424 | 10 545 | 24,87 | 57 | 40,8 |

0 130 288

Das nach der letzten Stufe gewonnene Enzympräparat kann für technische Zwecke eingesetzt werden. Durch weitere chromatographische Verfahren, z. B. Ionenaustauschchromatographie an Amberlite CG50I, Gelfiltration an Sephacryl S 200 oder hydrophobe Chromatographie an Phenyl-Sepharose CL-4B läßt sich das Enzym weiter aufreinigen bis zu einer spezifischen Aktivität von 110 U/mg. Das Substratspektrum wird dadurch nicht verändert. Das Molekulargewicht der D-2-Hydroxy-4-methylpentansäure-Dehydrogenase wurde durch Gelfiltration an Sephacryl S 200 superfine zu 73 000 ± 10 000 Dalton nach der Methode von Andrews (Meth. Biochemical Analysis, Vol. 18, 1 bis 53 (1970)) bestimmt. Als Eichsubstanzen wurden Dextran-blau, Ferritin, Aldolase, Rinderserumalbumin, Ovalbumin, Chymotrysinogen A und Cytochrom C verwendet.

Die erfindungsgemäße D-2-Hydroxy-4-methylpentansäure-Dehydrogenase kann einerseits zur Herstellung von D-2-Hydroxycarbonsäuren und andererseits zusammen mit einer L-Aminosäure-Dehydrogenase zur Umwandlung von D-2-Hydroxycarbonsäuren in die entsprechenden optisch aktiven L-Aminosäuren verwendet werden. Außerdem kann das neue Enzym aber auch dazu dienen, auf enzymatischem Wege in wässrigen Lösungen die Konzentrationen der als Substrate umgesetzten 2-Ketocarbonsäuren oder D-2-Hydroxycarbonsäuren zu bestimmen. Wenn die Substratkonzentration klein ist gegenüber dem $K_M$, hängt die Reaktionsgeschwindigkeit von der Substratkonzentration nach erster Ordnung ab. Die Bestimmung ist sehr einfach durchzuführen, es wird die Änderung der Extinktion von NADH bei 340 nm verfolgt und dann die gesuchte Konzentration aus einer entsprechenden Eichkurve abgelesen.

Die Stereoselektivität der enzymatischen Umsetzung wurde in einem Enzymmembranreaktor überprüft. Für die Regenerierung des bei der Reduktion der 2-Ketocarbonsäure verbrauchten NADH wird dabei eine Formiatdehydrogenase in einem Natriumformiatpuffer eingesetzt und anstelle des nativen NADH ein an Polyethylenglykol (mittleres Molgewicht 20 000) kovalent gebundenes NADH verwendet (Herstellung gemäß DE-PS 28 41 414). Die folgenden Ketosäuren wurden im Reaktor einzeln umgesetzt : 2-Keto-4-methylpentansäure (Natriumsalz), 2-Keto-4-(methylmercapto)-buttersäure (Natriumsalz) und Phenylpyruvat (Natriumsalz). Der den Membranreaktor verlassende Produktstrom wurde kontinuierlich in einem Polarimeter analysiert. Die erhaltenen Signale wurden anhand von Eichkurven ausgewertet, die mit optisch aktiven 2-Hydroxysäuren aufgenommen worden waren. Tabelle 3 zeigt, daß unter den gewählten Bedingungen sehr hohe Umsätze erreicht und nur die D-Isomeren der 2-Hydroxycarbonsäuren beobachtet werden. Die Resultate zeigen weiterhin, daß das erfindungsgemäße Enzym molekulargewichtsvergrößertes NADH als Substrat akzeptiert.

Tabelle 3 : Reduktion von 2-Ketocarbonsäuren

| Substrat | Drehwert der Produktions- lösung | Stereo- isomeres | Produktkon- zentration (% Umsatz) |
|---|---|---|---|
| 2-Keto-4-methylpentansäure | $-0,047^{\circ}$ | D | 7,0 mM (100 %) |
| 2-Keto-4-(methylmercapto)- buttersäure | $-0,071^{\circ}$ | D | 6,9 mM (98 %) |
| 2-Keto-3-phenylpropionsäure | $-0,115^{\circ}$ | D | 7,0 mM (100 %) |

Die Erfindung wird in den nachfolgenden Beispielen näher erläutert :

Beispiel 1 : Screening auf D-2-Hydroxy-4-methylpentansäure-Dehydrogenase-Produzenten

Es wurde ein Screening unter 45 Stämmen der Gattungen Lactobacillus und Leuconostoc aus dem Bestand der Deutschen Sammlung von Mikroorganismen durchgeführt. Die Stämme wurden in dem im Katalog der DSM angegebenen Medium in Schüttelkolben angezogen, die Zellen nach 20 bis 25 Stunden geerntet und anschließend durch Behandlung mit Ultraschall aufgeschlossen. Die unlöslichen Zelltrümmer wurden abzentrifugiert und der klare Überstand als Rohextrakt verwendet. Die Enzymaktivität wurde in einem photometrischen Test bestimmt, der Testansatz (3,00 ml) hatte folgende Zusammensetzung : 0,235 mM NADH, 0,79 mM 2-Keto-4-methylpentansäure und 0,1 M Phosphatpuffer für pH 7,0. Der Testansatz wurde 10 Minuten bei 30 °C inkubiert und danach die Reaktion durch Zugabe limitierender Mengen des Enzyms gestartet.

Die Enzymaktivität wurde durch Abnahme der Extinktion von NADH bei 340 nm gemessen. Abgezogen wurde ein Nullwert, den man erhielt, wenn der Test ohne 2-Keto-4-methylpentansäure ablief. Die Enzymaktivität wird in internationalen Einheiten (Units) angegeben, wobei 1 U den Verbrauch von

1 µMol NADH pro Minute und ml bedeutet.

Der geschilderte Test erlaubt keine Aussage darüber, welche Stereospezifität die NADH-abhängige 2-Hydroxy-4-methylpentansäure-Dehydrogenase besitzt. Das Suchprogramm wurde auf diese Weise durchgeführt, da sowohl an der D-spezifischen wie an der L-spezifischen Dehydrogenase Interesse bestand.

Eine Differenzierung zwischen den beiden Aktivitäten war möglich durch Verfolgen der Umsetzung von D-2-Hydroxy-4-methylpentansäure mit NAD$^+$, die nur von einer D-2-Hydroxy-4-methylpentansäure-Dehydrogenase katalysiert wird, während die L-2-Hydroxy-4-methylpentansäure-Dehydrogenase mit diesem Substrat inaktiv ist.

Dazu wurde folgender Testansatz verwendet : 3,0 mM NAD$^+$, 6,3 mM D-2-Hydroxy-4-methylpentansäure und 0,1 M Phosphatpuffer für pH 8,0. Der Testansatz (3,00 ml) wurde 10 Minuten bei 30 °C inkubiert und danach die Reaktion durch Zugabe limitierender Mengen des Enzyms gestartet. Abgezogen wurde der Nullwert, den man erhielt, wenn der Test ohne die D-2-Hydroxy-4-methylpentansäure ablief. In der obigen Tabelle 1 sind die sechs Stämme aufgeführt, die das erfindungsgemäße Enzym, die D-2-Hydroxy-4-methylpentansäure-Dehydrogenase enthalten, zusätzlich sind dort die Reaktionsgeschwindigkeiten der enzymatisch katalysierten Umsetzungen mit weiteren 2-Ketocarbonsäuren angegeben. Die Enzymaktivität wurde in dem oben beschriebenen Testansatz ermittelt, indem die 2-Keto-4-methylpentansäure jeweils durch eine andere Ketosäure, wie in Tabelle 1 angegeben, ersetzt wurde. Lactobacillus casei ssp. pseudoplantarum (DSM 20008) zeigte dabei das breiteste Substratspektrum.

Beispiel 2 : Abhängigkeit der Reaktionsgeschwindigkeit der enzymatisch katalysierten Umsetzung vom pH-Wert

Die Reaktionsgeschwindigkeit der Reduktion von 2-Keto-4-methylpentansäure zu D-2-Hydroxy-4-methylpentansäure in Gegenwart der D-2-Hydroxy-4-methylpentansäure-Dehydrogenase wurde in Abhängigkeit vom pH-Wert der Reaktionslösung untersucht. Der Testansatz (3,00 ml) hatte folgende Zusammensetzung : 0,1 mM NADH, 0,7 mM 2-Keto-4-methylpentansäure, limitierende Mengen an Enzym, 0,1 M Puffer verschiedener Zusammensetzung und verschiedener pH-Werte, wie in Abbildung I angegeben. In Abbildung I ist die Reaktionsgeschwindigkeit (ausgedrückt als Enzymaktivität in U/ml) als Funktion des pH-Wertes aufgetragen. Das Enzym zeigt ein relativ breites pH-Optimum zwischen pH 5,5 und pH 7 für die Reduktionsreaktion.

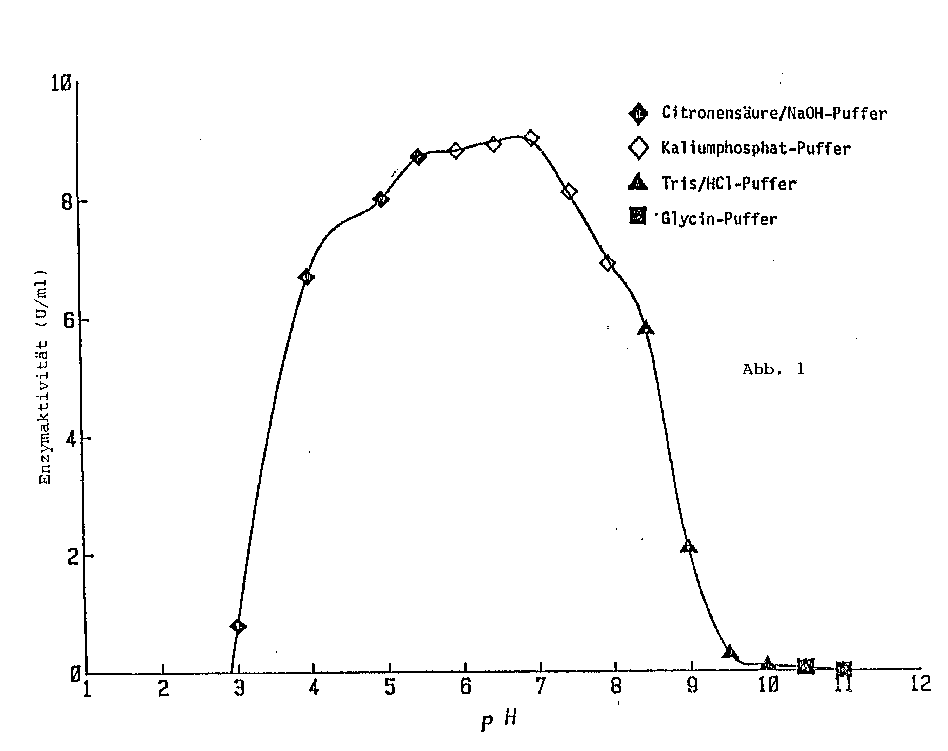

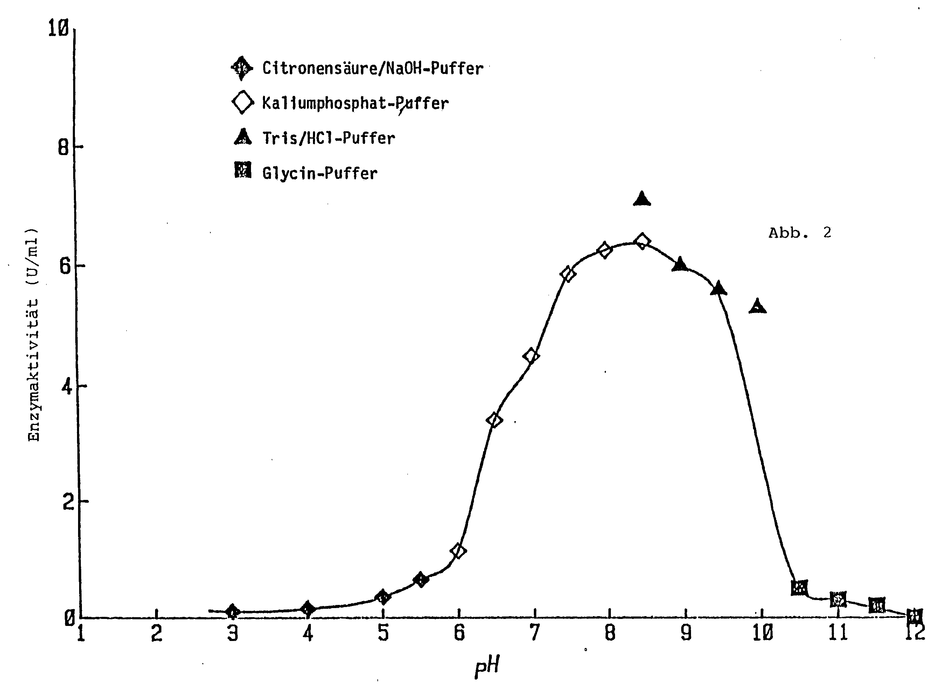

Die Reaktionsgeschwindigkeit der Dehydrogenierung von 2-Hydroxy-4-methylpentansäure zur 2-Keto-4-methylpentansäure, katalysiert durch die D-2-Hydroxy-4-methylpentansäure-Dehydrogenase, wurde gleichfalls in Abhängigkeit vom pH-Wert untersucht. Der Testansatz (3,00 ml) hatte folgende Zusammensetzung : 4,5 mM NAD$^+$, 12 mM D,L-2-Hydroxy-4-methylpentansäure, limitierende Mengen von Enzym, 0,1 M Puffer unterschiedlicher Zusammensetzung, wie in Abbildung 2 angegeben. Die Reaktionsgeschwindigkeit (ausgedrückt als Enzymaktivität in U/ml) der Dehydrogenierungsreaktion zeigt ein Optimum im Bereich pH 8 bis pH 9.

Beispiel 3 : Lagerstabilität der D-2-Hydroxy-4-methylpentansäure-Dehydrogenase in Abhängigkeit vom pH-Wert

D-2-Hydroxy-4-methylpentansäure-Dehydrogenase wurde in 0,1 M Puffer unterschiedlicher Zusammensetzung bei einer Proteinkonzentration von 0,03 mg/ml für 7 Tage bei 4 °C inkubiert. Danach wurde die Restaktivität, wie in Beispiel 2 beschrieben, bestimmt. Dabei zeigte sich eine außergewöhnliche pH-Stabilität im Bereich von pH 3,5 bis 10,0. Nach 24 Tagen waren noch > 95 % der Aktivität nachweisbar.

Beispiel 4 : Abhängigkeit der Reaktionsgeschwindigkeit von den Substratkonzentrationen

Die Abhängigkeit der Reaktionsgeschwindigkeit bei der Reduktion von 2-Keto-4-methylpentansäure zu D-2-Hydroxy-4-methylpentansäure von der Konzentration des Coenzyms NADH wurde in folgendem Testansatz untersucht : 0,1 M Phosphatpuffer für pH 7,0, 0,7 mM 2-Keto-4-methylpentansäure, limitierende Mengen an Enzym (angereichtes Präparat, nach DEAE-Cellulose-Chromatographie, siehe Tabelle 2) ; die NADH-Konzentration im Testansatz wurde im Bereich von 0,003 bis 0,33 mM variiert. Es zeigte sich, daß die optimale Reaktionsgeschwindigkeit bei 0,10 mM erreicht wird. Der $K_M$-Wert für NADH beträgt 0,010 mM. NADPH kann das Coenzym NADH nicht ersetzen, dies wurde bis zu einer Testkonzentration von 0,2 mM überprüft.

Die Reaktionsgeschwindigkeit bei der Reduktion verschiedener 2-Ketocarbonsäuren wurde auch in Abhängigkeit von der Ketosäurekonzentration untersucht. Dazu wurde folgender Testansatz verwendet : 0,1 M Phosphatpuffer für pH 7,0, 0,1 mM NADH und limitierende Mengen an Enzym (angereichtes Präparat nach DEAE-Cellulose-Chromatographie, siehe Tabelle 2). Die Ketocarbonsäurekonzentration wurde jeweils im Bereich von 0,01 bis maximal 60 mM variiert. Die Anfangsreaktionsgeschwindigkeit, Änderung der Extinktion bei 340 nm/min, wurde durch nicht-lineare Regression der Michaelis-Menten-Gleichung ausgewertet. Die gefundenen kinetischen Konstanten $K_M$ und $V_{max}$ sind in Tabelle 4 zusammengefaßt.

8

Tabelle 4 : Substratspezifität der D-2-Hydroxy-4-methylpentansäure-Dehydrogenase aus Lactobacillus casei ssp. pseudoplantarum

| Substrat | max. Anfangs-Reaktions-Geschwindigkeit $V_{max}$ ($\mu$Mol $\times$ min$^{-1}$ $\times$ mg$^{-1}$) | $K_M$-Wert (M) |
|---|---|---|
| 2-Ketobuttersäure | 45,9 | $1,7 \times 10^{-3}$ |
| 2-Ketopentansäure | 59,1 | $1,1 \times 10^{-4}$ |
| 2-Ketohexansäure | 58,4 | $1,1 \times 10^{-4}$ |
| 2-Ketooctansäure | 54,0 | $3,1 \times 10^{-4}$ |
| 2-Keto-3-methyl-buttersäure | 16,9 | $4,8 \times 10^{-3}$ |
| 2-Keto-3-methyl-pentansäure | 26,2 | $2,2 \times 10^{-3}$ |
| 2-Keto-4-methyl-pentansäure | 27,4 | $6,0 \times 10^{-5}$ |
| 2-Keto-4-(methylmercapto)-buttersäure | 82,1 | $6,2 \times 10^{-4}$ |
| 2-Keto-3-mercaptopropionsäure | 104,5 | $4,0 \times 10^{-4}$ |
| 2-Keto-3-phenylpropionsäure | 304,4 | $1,5 \times 10^{-4}$ |

Die Abhängigkeit der Reaktionsgeschwindigkeit bei der Dehydrogenierung von D-2-Hydroxy-4-methylpentansäure von der NAD$^+$-Konzentration wurde in folgendem Testansatz untersucht : 0,1 M Phosphatpuffer für pH 8,0, 12 mM, D,L-2-Hydroxy-4-methylpentansäure, limitierende Mengen an Enzym. Die NAD$^+$-Konzentration wurde im Bereich von 0,10 bis 10 mM variiert und die Zunahme der Extinktion durch das bei der Reaktion entstehende NADH bei 340 nm gemessen. Es zeigte sich, daß der optimale Umsatz bei einer Konzentration von 4,5 mM erreicht wird. Der $K_M$-Wert für NAD$^+$ beträgt 0,45 mM. NADP kann das Coenzym NAD$^+$ nicht ersetzen, dies wurde bis zu einer Testkonzentration von 10 mM überprüft.

Die Abhängigkeit der Reaktionsgeschwindigkeit bei der Dehydrogenierung von D-2-Hydroxycarbonsäuren von der Konzentration verschiedener 2-Hydroxycarbonsäuren wurde in folgendem Testansatz untersucht : 0,1 M Phosphatpuffer für pH 8,0, 4,5 mM NAD$^+$ und limitierende Mengen an Enzym. Die Konzentration der D-2-Hydroxycarbonsäuren wurde im Bereich von 0,1 bis maximal 30 mM variiert. Sofern keine chirale D-2-Hydroxycarbonsäure zur Verfügung stand, wurde das Racemat eingesetzt. Das bei der Reaktion entstehende NADH wurde bei 340 nm gemessen. Die Anfangsreaktionsgeschwindigkeit wurde nach Michaelis-Menten ausgewertet und die kinetischen Konstanten $V_{max}$ und $K_M$ durch nichtlineare Regression ermittelt. Die gefundenen kinetischen Konstanten sind in Tabelle 5 zusammengefaßt.

Tabelle 5 : Substratspezifität der D-2-Hydroxy-4-methylpentansäure-Dehydrogenase aus Lactobacillus casei ssp. pseudoplantarum

| Substrat | max. Anfangs-Reaktions-Geschwindigkeit $V_{max}$ ($\mu$Mol $\times$ min$^{-1}$ $\times$ mg$^{-1}$) | $K_M$-Wert (M) |
|---|---|---|
| D,L-2-Hydroxy-pentansäure | 10,8 | $1,4 \times 10^{-3}$ |
| D,L-2-Hydroxy-hexansäure | 11,3 | $9,3 \times 10^{-4}$ |
| D,L-2-Hydroxy-octansäure | 10,0 | $6,2 \times 10^{-4}$ |
| D,L-2-Hydroxy-4-methylpentansäure | 20,2 | $2,8 \times 10^{-3}$ |
| D-2-Hydroxy-4-methylpentansäure | 20,2 | $1,4 \times 10^{-3}$ |
| D,L-2-Hydroxy-4-(methylmercapto)-buttersäure | 2,6 | $3,0 \times 10^{-3}$ |
| D,L-2-Hydroxy-3-phenylpropionsäure | 32,6 | $2,1 \times 10^{-3}$ |

0 130 288

**0 130 288**

Beispiel 5 : Stereospezifität der Reduktion von 2-Keto-4-methylpentansäure und anderen 2-Ketocarbonsäuren mit der erfindungsgemäßen Dehydrogenase aus Lactobacillus casei ssp. pseudoplantarum (DSM 20008)

Eine kontinuierliche Synthese von D-2-Hydroxycarbonsäuren aus den entsprechenden 2-Ketocarbonsäuren ist in einem Enzymmembranreaktor unter Verwendung von molekulargewichtsvergrößertem, an Polyethylenglycol gebundenem NADH möglich. Das PEG-NADH wird gemäß DE-PS 28 41 414 hergestellt. Das modifizierte Coenzym und die eingesetzten Enzyme, Formiat-Dehydrogenase (Präparat gemäß Kroner et al. (1982), J. Chem. Tech. Biotechnol. 32, 130-137) und die D-2-Hydroxy-4-methylpentansäure-Dehydrogenase, werden durch eine Ultrafiltrationsmembran YM 5 (Produkt der Firma Amicon, Witten) im Reaktor zurückgehalten, während die niedermolekularen Bestandteile der Reaktionslösung, die gebildete optisch aktive 2-Hydroxycarbonsäure, gegebenenfalls nicht umgesetztes Substrat (optisch inaktive Ketosäure !) und der verwendete Puffer laufend aus dem Reaktor entfernt werden. Das Reaktorvolumen wurde konstant gehalten, indem im gleichen Maß aus einem Reservoir Ketosäure und Natriumformiatpuffer nachdosiert wurden, in dem das Ultrafiltrat den Reaktor verließ. Das Reaktorvolumen betrug 10 ml, die Konzentration an PEG-NADH 0,20 mM, es wurden 9 U D-2-Hydroxy-4-methylpentansäure-Dehydrogenase (27 U/mg) und 30 U Formiat-Dehydrogenase in den Reaktor eingespült. Die Substratlösung enthielt 300 mM Natriumformiatpuffer vom pH 7,0 und 7,0 mM der entsprechenden 2-Ketocarbonsäure. Die Reaktionslösung wurde mit einer Schlauchpumpe laufend mit 30 ml/Stunde über die Membran gepumpt. Etwa 3 ml Ultrafiltrat wurden pro Stunde erhalten. Das Ultrafiltrat wurde kontinuierlich durch eine Polarimeterküvette (Perkin Elmer Typ 241) geleitet. Die Messungen wurden bei 463 nm bei 25 °C durchgeführt. Aus dem gemessenen Drehwert wurde die Produktkonzentration nach einer Eichkurve ermittelt. Die Eichkurven wurden mit optisch aktiven 2-Hydroxycarbonsäuren in 300 mM Natriumformiatpuffer vom pH 7,0 aufgenommen. Dazu wurden die folgenden Präparate verwendet : L-Phenylmilchsäure (Firma Sigma, München), L-2-Hydroxy-4-methylpentansäure (Firma Sigma, München) und D-2-Hydroxy-4-(methylmercapto)-buttersäure (Firma Degussa, Hanau). Die Umsetzung im Membranreaktor wurde über 15 Stunden verfolgt. Die erhaltenen stationären Werte sind in der obigen Tabelle 3 zusammengefaßt.

Mit dem Substrat 2-Keto-4-methylpentansäure wurde in gleicher Weise die Stereoselektivität der von den anderen in der obigen Tabelle 1 aufgeführten Mikroorganismen produzierten 2-Hydroxy-4-methylpentansäure-Dehydrogenasen überprüft. Dabei wurden die Rohextrakte nach Beispiel 1 als Enzymquelle eingesetzt. Es wurde nur die Bildung der D-2-Hydroxy-4-methylpentansäure beobachtet bei Umsätzen zwischen 96 und 100 %.

**Patentansprüche**

1. Mikrobiologisch hergestellte D-2-Hydroxy-4-methylpentansäure-Dehydrogenase, gekennzeichnet durch folgende Eigenschaften :

a) katalysiert in Abhängigkeit von dem Coenzym Nicotin-amid-adenin-dinucleotid (NAD⁺) die Dehydrogenierung von D-2-Hydroxy-4-methylpentansäure zu 2-Keto-4-methylpentansäure,

b) katalysiert in Abhängigkeit von dem Coenzym NAD⁺ die Dehydrogenierung weiterer D-2-Hydroxycarbonsäuren zu den entsprechenden 2-Ketocarbonsäuren und zeigt insbesondere zusätzliche Aktivität gegenüber D-2-Hydroxy-pentansäure, D-2-Hydroxy-hexansäure, D-2-Hydroxy-octansäure, D-2-Hydroxy-4(methylmercapto)-buttersäure und D-2-Hydroxy-3-phenylpropionsäure,

c) katalysiert die stereoselektive Reduktion der 2-Keto-4-methylpentansäure zu D-2-Hydroxy-4-methylpentansäure mit NADH als Coenzym,

d) katalysiert die stereoselektive Reduktion weiterer 2-Ketocarbonsäuren, insbesondere von 2-Ketobuttersäure, 2-Ketopentansäure, 2-Keto-3-methylbuttersäure, 2-Ketohexansäure, 2-Keto-3-methylpentansäure, 2-Ketooctansäure, 2-Keto-3-mercaptopropionsäure, 2-Keto-4-(methylmercapto)-buttersäure und 2-Keto-3-phenylpropionsäure zu den entsprechenden D-2-Hydroxycarbonsäuren mit NADH als Coenzym,

e) einen optimalen pH-Bereich für die Dehydrogenierungsreaktion von 7,5 bis 9,0,

f) einen optimalen pH-Bereich für die Reduktionsreaktion von 5,5 bis 7,0 und

g) einen pH-Stabilitätsbereich von 3,5 bis 10

und erhältlich durch Züchtung eines der Stämme Lactobacillus casei ssp. pseudoplantarum (DSM 20008), Lactobacillus casei ssp. alactosus (DSM 20020), Lactobacillus casei ssp. rhamnosus (DSM 20178), Leucostonoc oenos (DSM 20252), Leuconstonoc lactis (DSM 20202) oder Leucostonoc mesenteroides (DSM 20343) und Gewinnung daraus.

2. Verfahren zur Gewinnung der D-2-Hydroxy-4-methylpentansäure-Dehydrogenase gemäß Anspruch 1, dadurch gekennzeichnet, daß man Lactobacillus casei ssp. pseudoplantarum (DSM 20008), Lactobacillus casei ssp. alactosus (DSM 20020), Lactobacillus casei ssp. rhamnosus (DSM 20178), Leuconostoc oenos (DSM 20252), Leuconostoc lactis (DSM 20202) oder Leuconostoc mesenteroides (DSM 20343) in einem wässrigen Nährmedium züchtet, das eine Quelle für Kohlenstoff, Stickstoff, Vitamine, gegebenenfalls Wuchsstoffe, sowie Mineralsalze enthält und zu Beginn der Züchtung einen pH-Wert von 6,5

12

aufweist, nach beendeter Züchtung die Zellen durch Zentrifugation erntet, sie in einer auf pH 7 gepufferten Suspension aufschließt und das Enzym aus dem Rohextrakt in an sich bekannter Weise weiter anreichert.

3. Verwendung der D-2-Hydroxy-4-methylpentansäure-Dehydrogenase gemäß Anspruch 1 zur enzymatisch Katalysierten Umwandlung von D-2-Hydroxy-4-methylpentansäure, D-2-Hydroxy-pentansäure, D-2-Hydroxy-hexansäure, D-2-Hydroxy-octansäure, D-2-Hydroxy-4-(methylmercapto)-buttersäure oder D-2-Hydroxy-3-phenylpropionsäure in die entsprechenden 2-Ketocarbonsäuren bzw. von 2-Keto-4-methylpentansäure, 2-Ketobuttersäure, 2-Ketopentansäure, 2-Keto-3-methylbuttersäure, 2-Ketohexansäure, 2-Keto-3-methylpentansäure, 2-Ketooctansäure, 2-Keto-3-mercaptopropionsäure, 2-Keto-4-(methylmercapto)-buttersäure und 2-Keto-3-phenylpropionsäure in die entsprechenden D-2-Hydroxycarbonsäuren.


**Claims**

1. Microbiologically prepared D-2-hydroxy-4-methyl pentanoic acid dehydrogenase, characterized by the following properties :

a) catalyzes in dependence upon the coenzyme nicotinamide adenine dinucleotide (NAD*) the dehydrogenation of D-2-hydroxy-4-methylpentanoic acid to 2-keto-4-methyl pentanoic acid,

b) catalyzes in dependence upon the coenzyme NAD* the dehydrogenation of other D-2-hydroxycarboxylic acids to the corresponding 2-ketocarboxylic acids and, in particular, shows additional activity with respect to D-2-hydroxypentanoic acid, D-2-hydroxyhexanoic acid, D-2-hydroxyoctanoic acid, D-2-hydroxy-4-(methylmercapto)-butyric acid and D-2-hydroxy-3-phenylpropionic acid,

c) catalyzes the stereoselective reduction of 2-keto-4-methylpentanoic acid to D-2-hydroxy-4-methylpentanoic acid with NADH as coenzyme,

d) catalyzes the stereoselective reduction of other 2-ketocarboxylic acids, particularly 2-ketobutyric acid, 2-ketopentanoic acid, 2-keto-3-methylbutyric acid, 2-ketohexanoic acid, 2-keto-3-methylpentanoic acid, 2-ketooctanoic acid, 2-keto-3-mercaptopropionic acid, 2-keto-4-(methylmercapto)-butyric acid and 2-keto-3-phenylpropionic acid, to the corresponding D-2-hydroxycarboxylic acids with NADH as coenzyme,

e) an optimal pH range for the dehydrogenation reaction of 7.5 to 9.0,

f) an optimal pH range for the reduction reaction of 5.5 to 7.0 and

g) a pH stability range of 3.5 to 10,

and obtainable by culture of one of the strains Lactobacillus casei ssp. pseudoplantarum (DSM 20008), Lactobacillus casei ssp. alactosus (DSM 20020), Lactobacillus casei ssp. rhamnosus (DSM 20178), Leuconostoc oenos (DSM 20252), Leuconostoc lactis (DSM 20202) or Leuconostoc mesenteroides (DSM 20343) and recovery therefrom.

2. A process for recovery of the D-2-hydroxy-4-methyl pentanoic acid dehydrogenase claimed in Claim 1, characterized in that Lactobacillus casei ssp. pseudoplantarum (DSM 20008), Lactobacillus casei ssp. alactosus (DSM 20020), Lactobacillus casei spp. rhamnosus (DSM 20178), Leuconostoc oenos (DSM 20252), Leuconostoc lactis (DSM 20202) or Leuconostoc mesenteroides (DSM 20343) is cultured in an aqueous nutrient medium which contains a source for carbon, nitrogen, vitamins, optionally growth promotors and mineral salts and which has a pH value of 6.5 at the beginning of the culture process, the cells are collected by centrifuging on completion of the culture process, digested in a suspension buffered to pH 7 and the enzyme is further enriched from the crude extract by methods known per se.

3. The use of the D-2-hydroxy-4-methylpentanoic acid dehydrogenase claimed in Claim 1 for the enzymatically catalyzed conversion of D-2-hydroxy-4-methylpentanoic acid, D-2-hydroxypentanoic acid, D-2-hydroxyhexanoic acid, D-2-hydroxyoctanoic acid, D-2-hydroxy-4-(methylmercapto)-butyric acid or D-2-hydroxy-3-phenylpropionic acid into the corresponding 2-ketocarboxylic acids or of 2-keto-4-methylpentanoic acid, 2-ketobutyric acid, 2-ketopentanoic acid, 2-keto-3-methylbutyric acid, 2-ketohexanoic acid, 2-keto-3-methylpentanoic acid, 2-ketooctanoic acid, 2-keto-3-mercaptopropionic acid, 2-keto-4-(methylmercapto)-butyric acid and 2-keto-3-phenylpropionic acid into the corresponding D-2-hydroxycarboxylic acids.


**Revendications**

1. Déhydrogénase de l'acide D-2-hydroxy-4-méthylpentanique fabriquée par un procédé microbiologique, caractérisée en ce qu'elle possède les propriétés suivantes :

a) elle catalyse en fonction de la présence du coenzyme nicotinamide-adénine-dinucléotide (NAD+), la déhydrogénation de l'acide D-2-hydroxy-4-méthylpentanique en acide 2-céto-4-méthylpentanique,

b) elle catalyse, en fonction de la présence du coenzyme NAD+, la déhydrogénase d'autres acides D-2-hydroxycarboxyliques en acides 2-cétocarboxyliques correspondants, et montre en particulier une activité supplémentaire par rapport aux acides D-2-hydroxy-pentanique, D-2-hydroxy-hexanique, D-2-hydroxy-octanique, D-2-hydroxy-4-(méthylmercapto)-butyrique, et D-2-hydroxy-3-phénylpropionique,

c) elle catalyse la réduction stéréosélective de l'acide 2-céto-4-méthylpentanique en acide D-2-hydroxy-4-méthylpentanique avec NADH comme coenzyme,

d) elle catalyse la réduction stéréosélective d'autres acides 2-cétocarboxyliques, en particulier des acides 2-cétobutyrique, 2-cétopentanique, 2-céto-3-méthylbutyrique, 2-cétohexanique, 2-céto-3-méthylpentanique, 2-céto-octanique, 2-céto-3-mercaptopropionique, 2-céto-4-(méthylmercapto)-butyrique, et 2-céto-3-phénylpropionique, en les acides D-2-hydroxycarboxyliques correspondants, avec NADH comme coenzyme,

e) une zone de pH optimum pour la réaction de déhydrogénation de 7,5 à 9,

f) une zone de pH optimum pour la réaction de réduction, de 5,5 à 7, et,

g) une zone de stabilité du pH de 3,5 à 10,

et peut être obtenue par culture d'une des souches lactobacillus casei ssp. pseudoplantarum (DSM 20008), Lactobacillus casei ssp. alactosus (DSM 20020), Lactobacillus casei ssp. rhamnosus (DSM 20178), Leucostonoc oenos (DSM 20252), Leuconstonoc lactis (DSM 20202) ou Leucostonoc mesenteroïdes (DSM 20343) et récupération à partir de ces cultures.

2. Procédé pour l'obtention de la déhydrogénase de l'acide D-2-hydroxy-4-méthylpentanique suivant la revendication 1, caractérisé en ce que l'on cultive le Lactobacillus casei ssp. pseudoplantarum (DSM 20008), Lactobacillus casei ssp. alactosus (DSM 20020), Lactobacillus casei ssp. rhamnosus (DSM 20178), Leuconostic oenos (DSM 20252), Leuconostoc lactis (DSM 20202) et Leuconostoc mesenteroïdes (DSM 20343) dans un milieu nutritif aqueux, qui contient une source de carbone, de l'azote, des vitamines, éventuellement des substances de croissance, ainsi que des sels minéraux et qui présente au début de la culture un pH de 6,5, et que la culture une fois terminée, on récolte les cellules par centrifugation, les brise dans une suspension tamponnée à pH 7, et enrichit l'enzyme à partir de l'extrait brut d'une façon connue en soi.

3. Utilisation de la déhydrogénase de l'acide D-2-hydroxy-4-méthylpentanique suivant la revendication 1 pour la transformation avec catalyse enzymatique des acides D-2-hydroxy-4-méthylpentanique, D-2-hydroxy-pentanique, D-2-hydroxyhexanique, D-2-hydroxyoctanique, D-2-hydroxy-4-(méthylmercapto)-butyrique, D-2-hydroxy-3-phénylpropionique en les acides 2-cétocarboxyliques correspondants ou les acides 2-céto-4-méthylpentanique, 2-cétobutyrique, 2-céto-pentanique, 2-céto-3-méthylbutyrique, 2-cétohexanique, 2-céto-3-méthylpentanique, 2-cétooctanique, 2-céto-3-mercaptopropionique, 2-céto-4-(méthylmercapto)-butyrique, et 2-céto-3-phénylpropionique en les acides D-2-hydroxycarboxyliques correspondants.

14

Abb. 1

Abb. 2

0 130 288